# EUROPÄISCHE PATENTSCHRIFT

(11) **EP 3 675 937 B1**
(45) Veröffentlichungstag und Bekanntmachung des Hinweises auf die Patenterteilung: **01.10.2025**
(21) Anmeldenummer: 18773619.4
(22) Anmeldetag: 27.08.2018
(51) Int. Cl.: A61M 5/315, A61M 5/31, A61M 5/20, A61M 5/32

(54) **ANTRIEBSVORRICHTUNG FÜR INJEKTIONSGERÄTE**
DRIVE APPARATUS FOR INJECTION DEVICES
DISPOSITIF D'ENTRAÎNEMENT POUR APPAREILS D'INJECTION

(30) Priorität: 31.08.2017 CH 10792017
(43) Veröffentlichungstag der Anmeldung: 08.07.2020
(73) Patentinhaber: Ypsomed AG, 3401 Burgdorf (CH)
(72) Erfinder: WITTMANN, Jürgen, 3400 Burgdorf (CH); TSCHIRREN, Markus, 3400 Burgdorf (CH); KLÖTZLI, Urs, VIC 3071 Thornbury (AU)
(74) Vertreter: Meier Obertüfer, Jürg
(86) Internationale Anmeldenummer: PCT/IB2018/056478
(87) Internationale Veröffentlichungsnummer: WO 2019/043549

(56) Entgegenhaltungen:
- WO-A1-2005/115514
- WO-A1-2016/042075
- WO-A1-2016/042075
- WO-A1-2016/205962
- DE-A1- 102007 013 838
- US-A1- 2016 015 901

## Beschreibung

### TECHNISCHES GEBIET

Die vorliegende Erfindung betrifft das Gebiet der medizinischen Injektionsgeräte zur Verabreichung von flüssigen Substanzen, insbesondere von Medikamenten oder medizinischen Substanzen wie Insulin- und Hormonpräparationen. Die Erfindung bezieht sich auf eine platzsparende Antriebsvorrichtung mit einer Kolbenstange zum Vorschub eines Stopfens in einem Produktbehälter eines Injektionsgeräts.

### HINTERGRUND

Injektionsgeräte oder Injektionsvorrichtungen zum vereinfachten Verabreichen einer Substanz umfassen unter anderem so genannte Autoinjektoren, welche ein Energiespeicher- oder Antriebselement aufweisen, mit welchem die Ausschüttung automatisch, ohne extern von einem Benutzer zuzuführende oder aufzuwendende Kraft, durchgeführt werden kann. Das Energiespeicher- oder Antriebselement speichert vorteilhaft die vollständige für eine automatische Substanzabgabe erforderliche Energie in mechanischer Form. Ein solches Energiespeicher- oder Antriebselement kann beispielsweise eine Feder sein, welche in einem gespannten Zustand in das Injektionsgerät eingebaut wird und durch Entspannen Energie abgeben kann. Die Energieabgabe erfolgt vorteilhaft an eine Kolbenstange oder ein Druckelement, welches einen Stopfen in einen Produktbehälter einschieben kann. Optional kann das Energiespeicher- oder Antriebselement auch vorgesehen sein um den Vorgang des Einstechens einer Injektionsnadel zu automatisieren. Alternativ kann zu diesem Zweck ein weiteres separates Antriebselement vorgesehen sein, oder der Einstechvorgang erfolgt manuell, also durch einen Benutzer, ohne hierfür in dem Injektionsgerät gespeicherte Energie zu verwenden.

Das Injektionsgerät kann einen Produktbehälterhalter zur Aufnahme eines Produktbehälters umfassen, wobei in dem Produktbehälterhalter der Produktbehälter fest, insbesondere axial- und vorzugsweise drehfest gehalten werden kann. Der Produktbehälterhalter kann mit dem Gehäuse der Injektionsvorrichtung axial- und drehfest verbunden sein, oder bei einem Einstech- und/oder Nadelrückzugsvorgang relativ zum Gehäuse bewegbar sein. Der Produktbehälter kann eine Karpule oder eine aus dem Stand der Technik bekannte Fertigspritze mit einer damit unlösbar verbundenen Injektionsnadel sein. Der Produktbehälter weist einen hohlzylindrischen Produktbehälterabschnitt auf, der einen Kolben oder Stopfen verschiebbar lagert. Der Kolben kann mit dem Innenumfang des Produktbehälterabschnitts einen Dichtspalt bilden und mittels Kolbenstange in eine distale Richtung verschoben werden, um über die Injektionsnadel Produkt aus dem Produktbehälter abzugeben.

Das Injektionsgerät kann eine Nadelschutzhülse aufweisen, die nach erfolgter Injektion distal über das distale Ende der Injektionsnadel steht oder relativ zu dem Gehäuse unter Entspannung einer Nadelschutzhülsenfeder in diese Position verschoben wird, um den versehentlichen Zugriff auf die Injektionsnadel zu verhindern und dadurch ein Verletzungsrisiko zu verringern. Bei einem Autoinjektor kann die Nadelschutzhülse auch als Auslöseelement zum Auslösen der Produktausschüttung dienen, wobei die Nadelschutzhülse hierzu relativ zu dem Gehäuse in die proximale Richtung verschoben wird. Alternativ kann die Auslösung des Autoinjektors durch Betätigen eines Auslöseknopfs des Autoinjektors erreicht werden, wobei die Nadelschutzhülse vor dem Gebrauch des Autoinjektors zumindest als Sichtschutz dient.

Die Patentanmeldung EP 2781230 beschreibt einen Autoinjektor zum automatischen Ausschütten einer medizinischen Substanz mittels einer vorgespannten Ausschütt- oder Injektionsfeder welche über eine Kolbenstange einen Stopfen in eine Fertigspritze drückt. Die Injektionsvorrichtung umfasst weiter eine in einer Längsrichtung zwischen einer proximalen und einer distalen Position verschiebbare Nadelschutzhülse. Die Nadelschutzhülse ist mit einer Nadelschutzhülsenfeder als separatem Antriebselement gekoppelt, welche die Nadelschutzhülse nach erfolgter Substanzabgabe in die distale Position schiebt, in welcher sie die Nadel seitlich umgibt oder abschirmt. Ein bewegliches Anschlagelement als Rückkopplungsvorrichtung zur Erzeugung eines akustischen Signals nach Abgabe einer bestimmten Menge an Substanz wird durch die Nadelschutzhülsenfeder zu einem Anschlag hin beschleunigt. Eine zweite Rückkopplungsvorrichtung mit einem durch die Ausschüttfeder beschleunigten Anschlagelement signalisiert den Beginn der Substanzabgabe. Die Nadelschutzhülsenfeder ist in einem proximalen Teil des Autoinjektors angeordnet und umgibt die Kolbenstange in deren Ausgangsposition.

WO 2005/115514 A1 beschreibt ein Antriebselement in Form einer Kolbenstange mit, an einem distalen, dem Stopfen zugewandten Ende der Kolbenstange, einer konischen Spitze umgeben von einer ringförmigen Antriebsfläche. Der Kolben oder Stopfen einer Spritze weist auf seiner proximalen Seite eine zentrale Bohrung oder Ausnehmung mit Innengewinde auf, in welche die Spitze der Kolbenstage beim Ausschütten hineinragt. Die Bohrung des Stopfens ist umgeben von einer ringförmigen Auflagefläche welche beim Ausschütten mit der Antriebsfläche der Kolbenstange in Kontakt tritt. Eine proximale rotationssymmetrische Öffnung der Spritze ist trichterförmig ausgeweitet, ihre Innenfläche weist in einer die Längsachse umfassenden Ebene einen Minimalradius auf. Die Kombination von Kolbenstangenspitze als Zentrierhilfe mit der abgerundeten Innenform der Spritze erlaubt es, eine unzulängliche axiale Ausrichtung von Kolbenstange und Spritze beim Einführen Letzterer in die proximale Öffnung der Spritze zu kompensieren oder zu korrigieren. Die maximale radiale Breite der kreisringförmigen Antriebsfläche beträgt auf jeden Fall weniger als ein Fünftel des Gesamtdurchmessers der Kolbenstange.

DE 10 2007 013 838 A1 offenbart eine Injektionsvorrichtung mit einem Produktbehälter und einer proximal dieser angeordneten Funktionshülse, welche eine Kolbenstange zur Produktausschüttung umgibt. Die Kolbenstange weist einen hülsenförmigen Teil auf, der eine Vortriebsfeder umgibt, wobei sich die Vortriebsfeder distal an der Kolbenstange und proximal an einer Schalthülse abstützt. Während einem Auslösevorgang wird die Schalthülse freigegeben, so dass sich die Vortriebsfeder teilweise entspannen kann, wodurch die Karpule zusammen mit einem Karpulenhalter und der Funktionshülse in Einstechrichtung verschoben wird. Dadurch wird die Injektionsnadel über das distale Ende der Injektionsvorrichtung hervor geschoben und sticht in die Injektionsstelle ein.

WO 2016/205962 A1 offenbart einen Autoinjektor, welcher eine zweiteilige Kolbenstange umfasst, welche eine innere Gewindestange und ein äusseres hülsenförmiges Vortriebsglied beinhaltet, welches in Gewindeverbindung mit der Gewindestange ist. Die Gwindestange ist mit einer vorgespannten Spiralfeder verbunden, welche die für die Produktausschüttung notwendige Energie gespeichert hat.

WO 2016/042075 A1 offenbart einen elektrischen Autoinjektor mit einen elektrischen Motor, welcher innerhalb einer hülsenförmigen Kolbenstange angeordnet ist. Die Kolbenstange weist am distalen Ende einen Flansch auf, welcher einen Stopfen im Produktbehälter verschieben kann. Beim Ausschütten treibt der Motor ein Antriebsrad an, das auf seiner Aussenseite ein Gewindesegment aufweist, welches mit einem Innengewinde der Kolbenstange in Verbindung steht. Das drehende Antriebsrad bewirkt, dass die Kolbenstange in Ausschüttrichtung verschoben wird.

US 2016/0015901 A1 offenbart ein Injektionsgerät mit einer Kolbenstange, die am distalen Ende einen knopfförmigen Abschluss hat. Dieser wird bei der Montage mit einem Flansch verbunden. Der Flansch weist hierzu in der Mitte eine Öffnung auf, wobei das Material um die Öffnung flexibel ist, so dass zum Zusammenfügen der Kolbenstange mit dem Flansch die Öffnung aufgedehnt werden kann, so dass das knopfförmige Ende durch die Öffnung geführt werden kann.

Der Begriff "Produkt", "Medikament" oder "medizinische Substanz" umfasst im vorliegenden Zusammenhang jede fliessfähige medizinische Formulierung, welche geeignet ist zur kontrollierten Verabreichung mittels einer Kanüle oder Hohlnadel, beispielsweise eine Flüssigkeit, eine Lösung, ein Gel oder eine feine Suspension enthaltend einen oder mehrere medizinische Wirkstoffe. Ein Produkt oder Medikament kann also eine Zusammensetzung mit einem einzigen Wirkstoff oder eine vorgemischte oder co-formulierte Zusammensetzung mit mehreren Wirkstoffen aus einem einzelnen Behälter sein. Der Begriff umfasst insbesondere Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form. Der Begriff umfasst weiter auch Polysaccharide, Vakzine, DNS oder RNS oder Oligonukleotide, Antikörper oder Teile von Antikörpern sowie geeignete Basis-, Hilfs- und Trägerstoffe.

Der Begriff "distal" bezieht sich auf die Richtung, in die die Spitze der Injektionsnadel zeigt, und der Begriff "proximal" bezieht sich auf die Richtung, die der distalen Richtung entgegengesetzt ist.

### DARSTELLUNG DER ERFINDUNG

Es ist eine Aufgabe der Erfindung, ein Injektionsgerät mit einem reduzierten Platzbedarf und einem sicheren Antrieb mit ausreichend homogener Krafteinleitung auf einen verschiebbaren Stopfen zu schaffen. Diese Aufgabe wird gelöst durch ein Injektionsgerät nach Anspruch 1. Weitere vorteilhafte Ausgestaltungen ergeben sich aus den abhängigen Ansprüchen.

Ein erfindungsgemässes Injektionsgerät zur Verabreichung eines Produkts umfasst ein Gehäuse zur Aufnahme eines Produktbehälters mit einem Produktaufnahmeraum zur Aufnahme des Produktes. Im Produktbehälter ist ein Stopfen mit einer Auflagefläche zum Ausschütten des Produkts aus dem Produktaufnahmeraum in Richtung einer Längsachse der Injektionsvorrichtung bis zu einer Stopfenendposition verschiebbar. Der Produktbehälter weist proximal der Stopfenendposition an seiner engsten Stelle einen minimalen Produktbehälterquerschnitt in einer Ebene senkrecht zur Längsachse auf. Das Injektionsgerät umfasst weiter einen Antrieb zur Verschiebung des Stopfens, umfassend eine Kolbenstange mit einem Kolbenstangenquerschnitt senkrecht zur Längsachse und einer stopfenseitigen Antriebsfläche zum Antrieb des Stopfens durch kraftschlüssigen Kontakt mit dessen Auflagefläche. Das Injektionsgerät umfasst auch eine proximal des Produktbehälters im Gehäuse angeordnete Kolbenstangenführung mit einem Führungsquerschnitt senkrecht zur Längsachse zur Führung einer Bewegung der Kolbenstange in Richtung der Längsachse. Der Führungsquerschnitt ist an den Kolbenstangenquerschnitt angepasst oder darauf abgestimmt, und begrenzt eine Bewegung des Kolbenstangenquerschnitts in alle Richtungen senkrecht zur Längsachse auf ein fertigungstechnisches Minimum. Der Produktbehälter selbst führt die Bewegung der Kolbenstange in Richtung der Längsachse höchstens unvollständig oder unsymmetrisch, so dass der Kolbenstangenquerschnitt zumindest an einer seitlichen Bewegung in eine erste Verkippungsrichtung senkrecht zur Längsachse nicht durch den Produktbehälterquerschnitt gehindert wird. An einem distalen Ende der Kolbenstange ist eine Kolbenstangenzentrierhilfe zur Begrenzung der Bewegung des Kolbenstangenquerschnitts in die erste Verkippungsrichtung beziehungsweise zur Begrenzung einer Verkippung der Kolbenstange vorgesehen. Dadurch wirkt die Kolbenstangenzentrierhilfe auch einer rotationsasymmetrischen Antriebskraftverteilung über die Auflagefläche entgegen.

In einer vorteilhaften Variante der Erfindung wird von einem kreisrunden Produktbehälterquerschnitt mit einem ersten Durchmesser ausgegangen, wobei für andere, beispielsweise ovale Querschnittsformen die geometrischen Überlegungen betreffend Längsführung der Kolbenstange entsprechend differenziert anzupassen wären. Ebenso werden vorteilhafterweise kreisrunde Führungs- und Kolbenstangenquerschnitte mit einem zweiten beziehungsweise dritten Durchmesser angenommen, wobei auch eckige aufeinander abgestimmte Querschnittsformen denkbar sind, zumindest solange ein kleinster den Kolbenstangenquerschnitt umfassender Kreis innerhalb des Produktbehälterquerschnitts Platz findet. Mit den genannten Bezeichnungen ist der erste Durchmesser grösser als der zweite Durchmesser, und dieser wiederum nicht kleiner als der dritte Durchmesser. Bevorzugt ist der erste Durchmesser mindestens vier oder gar sieben Prozent grösser als der dritte Durchmesser. In einer anderen vorteilhaften Variante umfasst der Stopfen eine zentrale Ausnehmung innerhalb der Auflagefläche, bevorzugt eine kreisrunde Bohrung mit einem optionalen Innengewinde. Entsprechend ist die Auflagefläche vorteilhafterweise eine Kreisringfläche.

Erfindungsgemäss wird also eine Kolbenstange mit einem Maximalaussendurchmesser eingesetzt, welcher bevorzugt um mindestens vier Prozent kleiner ist als der Durchmesser des Produktbehälters. Die dadurch wegfallende Längsführung der Kolbenstange durch den Produktbehälter wird kompensiert durch eine Zentrierhilfe an der Kolbenstangenspitze, welche einer ungenauen oder unzureichenden Ausrichtung der Kolbenstange im Produktbehälter entgegenwirkt. Auf diese Weise kann im Gehäuse im Bereich der Kolbenstangenführung und des Antriebs Platz eingespart werden ohne das Risiko einer unvorhersehbaren oder gar unsicheren Abweichung der Ausrichtung der Kolbenstange von der Längsachse. Die Kolbenstangenzentrierhilfe ist bevorzugt aus demselben, unelastischen Material gefertigt wie die Kolbenstange.

In einer ersten bevorzugten Ausführungsform des Injektionsgeräts umfasst die Kolbenstangenzentrierhilfe einen Zentrierflansch zur Vergrösserung des Kolbenstangenquerschnitts. Der Zentrierflansch weist also einen maximalen Flanschdurchmesser auf, welcher grösser ist als ein Maximalaussendurchmesser der Kolbenstange und kleiner als ein Minimalinnendurchmesser des Produktbehälters. Bevorzugt ist der Zentrierflansch bündig mit der Antriebsfläche angeordnet, so dass auch die Kraftübertragungsfläche gegenüber einem Kolbenstangendistalabschluss ohne Zentrierflansch vergrössert ist.

In einer zweiten nicht erfindungsgemäßen Ausführungsform des Injektionsgeräts umfasst die Kolbenstangenzentrierhilfe nur einen Zentrierstumpf, insbesondere einen Kegel- oder Pyramidenstumpf. Der Zentrierstumpf weist an seiner Basis einen Basisdurchmesser auf welcher kleiner ist als der Durchmesser der Bohrung des Stopfens. Der Zentrierstumpf kann mit dem vorgenannten Zentrierflansch in einer weiteren bevorzugten erfindungsgemäßen Ausführungsform kombiniert werden.

Die Erfindung betrifft auch einen Antrieb für ein Injektionsgerät und eine Kolbenstange für einen Antrieb für ein Injektionsgerät. Für den Fachmann sind zudem direkt und offensichtlich weitere Ausführungsformen und Ausgestaltungen erkennbar, welche sich aus Kombinationen der beschriebenen Beispiele oder Kombinationen der beschriebenen Beispiele mit dem allgemeinen Fachwissen des Fachmanns ergeben. Die ursprünglichen Rückbezüge der abhängigen Ansprüche sollen keine weiteren Anspruchs- oder Merkmalskombinationen ausschliessen.

### FIGUREN

In Zusammenhang mit den angehängten Figuren werden nachfolgend bevorzugte Ausführungsformen der Erfindung beschrieben. Diese sollen grundsätzliche Möglichkeiten der Erfindung aufzeigen und keinesfalls einschränkend ausgelegt werden. Es zeigen
- Fig.1: eine Längsschnittansicht einer Injektionsvorrichtung,
- Fig.2: eine Schrägaufsicht einer ersten Ausführungsform mit einem Zentrierflansches,
- Fig.3: einen Längsschnitt der ersten Ausführungsform,
- Fig.4: eine Schrägaufsicht einer zweiten Ausführungsform mit einem Zentrierkonus,
- Fig.5: einen Längsschnitt der zweiten Ausführungsform,
- Fig.6: zwei Längsschnittansichten eines Autoinjektors in einem Initialzustand, und
- Fig.7: zwei Längsschnittansichten des Autoinjektors in einem ausgelösten Zustand.

Die Bezugszeichen und deren Bedeutung sind zusammengefasst in der Bezugszeichenliste. Im Allgemeinen bezeichnen dieselben Bezugszeichen dieselben Teile.

### FIGURENBESCHREIBUNG

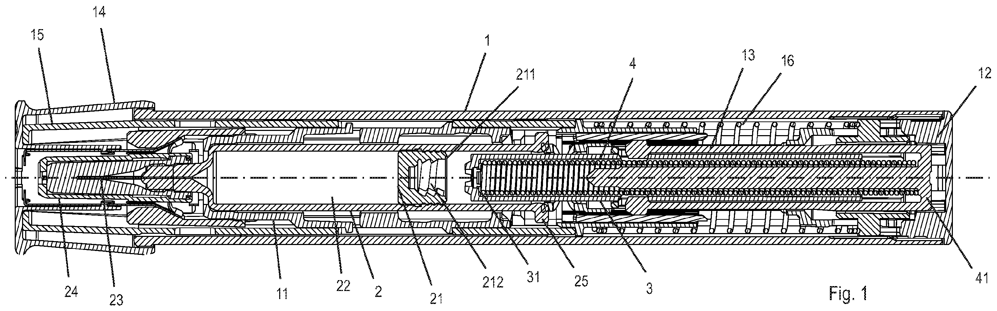

Fig.1 zeigt eine Längsschnittansicht einer Injektionsvorrichtung. Die Injektionsvorrichtung umfasst ein hülsenförmiges, insbesondere zylindrisches, Gehäuse 1, an welchem ein Produktbehälterhalter 11 und eine Endkappe 12 angeordnet sind. Der Produktbehälterhalter 11 und die Endkappe 12 können gehäusefest mit dem Gehäuse 1 verrastet, verklebt, verschweisst, verriegelt oder verschnappt sein, oder auch jeweils oder insgesamt einteilig mit dem Gehäuse 1 ausgebildet sein. In dem Produktbehälterhalter 11 kann ein Produktbehälter 2 aufgenommen und von diesem gehalten werden. Der Produktbehälter umfasst einen durch einen entlang einer Längsachse des Produktbehälters 2 verschiebbaren Stopfen 21 begrenzten, hohlzylindrischen Produktaufnahmeraum 22. Im Produktaufnahmeraum 22 ist bei Inbetriebnahme ein abzugebendes Produkt enthalten welches durch Verschiebung des Stopfens 21 in einer Verdrängungsrichtung von einer Stopfenstartposition in eine Stopfenendposition aus dem Produktaufnahmeraum 22 heraus verdrängt werden kann. Der Stopfen 21 weist auf einer der Verdrängungsrichtung abgewandter Seite eine ringförmige Auflagefläche 211 auf, welche eine zentrale Bohrung 212 oder Ausnehmung mit einem optionalen Innengewinde umgibt. Die Injektionsvorrichtung umfasst weiter einen Antrieb mit einer Kolbenstange 3 zur Bewegung des Stopfens 21 des Produktbehälters 2 in Verdrängungsrichtung. Die Kolbenstrange 3 ist ausserhalb des Produktbehälters 2 durch eine Kolbenstangenführung 13 in Richtung der Längsachse geführt. Die Kolbenstangenführung 13 kann ihrerseits in Richtung der Längsachse verschiebbar sein und eine die Kolbenstange 3 umgebende Hülse und/oder einen von der Kolbenstange 3 umgebenen Dorn umfassen. Die Kolbenstangenführung 13 kann gleichzeitig die Funktion eines Halteelements übernehmen, welches eine Bewegung der Kolbenstange 3 in Ausschüttrichtung verhindert und eine Injektionsfeder 4 in ihrem gespannten Zustand hält. Dazu kann die Kolbenstangenführung 13 zwei flexible Haltearme 6c mit einander gegenüberliegenden Eingriffselementen 6a aufweisen welche initial in Ausnehmungen 34 der Kolbenstange 3 eingreifen. Nach erfolgter Auslösung gleitet die Kolbenstange 3 an den flexibel gelagerten Eingriffselementen 6a entlang, so dass zwischen den Eingriffselementen 6a eine Linearausdehnung D13 des Querschnitts Q13 der Kolbenstangenführung 13 definiert ist welche gleich gross ist wie eine Linearausdehnung D3 eines Querschnitts Q3 der Kolbenstange 3 in dieselbe Richtung.

Der in Fig.1 dargestellte bevorzugte Produktbehälter ist eine aus Glas oder Kunststoff gefertigte Fertigspritze 2 mit einem Produktaufnahmeraum 22, aus welchem ein abzugebendes Produkt durch Verschiebung des Stopfens 21 in distale Längsrichtung der Fertigspritze 2 (in Fig.1 nach links) durch eine mit der Vorderseite der Fertigspritze 2 fest verbundene Injektionsnadel 23 abgegeben werden kann. Die Injektionsnadel 23 ist in dem in Fig. 1 gezeigten Ausgangszustand von einer Nadelschutzkappe 24 umgeben, in welcher ein elastisches, beispielsweise aus TPE oder aus Gummi gefertigtes Nadelschutzelement vorhanden ist. Die Nadelschutzkappe 24 kann zusammen mit dem Nadelschutzelement mittels eines darauf aufgesetzten Kappenabzugselements 14 abgezogen werden, um die Injektionsnadel 23 freizulegen. Die Injektionsnadel 23 ist weiter von einer relativ zum Gehäuse 1 axial verschiebbar gelagerten und in das Gehäuse 1 einschiebbaren Nadelschutzhülse 15 umgeben. Die Nadelschutzhülse 15 weist an ihrer distalen Stirnseite eine Durchtrittsöffnung auf, durch welche die Injektionsnadel 23 bei einer Relativbewegung von Nadelschutzhülse und Injektionsnadel hindurchtreten kann. Die Nadelschutzhülse 15 kann auch als Auslöseelement zum Auslösen der Produktausschüttung dienen, wobei die Nadelschutzhülse hierzu unter Spannung einer Nadelschutzhülsenfeder 16 relativ zu dem Gehäuse in die proximale Richtung verschoben wird. Am proximalen Ende der Fertigspritze 2 ist ein Flansch 25, der auch als Fingerflansch bezeichnet wird, angeordnet.

Der in Fig.1 dargestellte Antrieb umfasst weiter eine als Energiespeicher oder Antriebsmittel dienende Injektionsfeder 4 oder Ausschüttfeder in Form einer Druckfeder. Die Injektionsfeder 4 speichert bevorzugt mindestens die Energie für eine vollständige Ausschüttung des Produktes im Produktaufnahmeraum 22 und wird als derart vorgespannte Feder in die Injektionsvorrichtung eingesetzt. Die Injektionsfeder 4 ist innerhalb der Kolbenstange 3 angeordnet und stützt sich gegen ein stirnseitiges Abschlusselement 31 der Kolbenstange ab. In proximaler Richtung wird die Injektionsfeder 4 von einem proximalen Boden- oder Tellerelement 41 gehalten und während der Produktausschüttung gestützt. In Fig.1 ist das Bodenelement 41 einteilig mit der Kolbenstangenführung 13 ausgebildet und zur Erzeugung eines Startsignals in Längsrichtung bewegbar. Alternativ kann die Injektionsfeder die Form einer Wendelfeder oder Torsionsfeder annehmen, und/oder neben der Produktausschüttung auch für eine Bewegung des Produktbehälterhalters 11 verantwortlich sein.

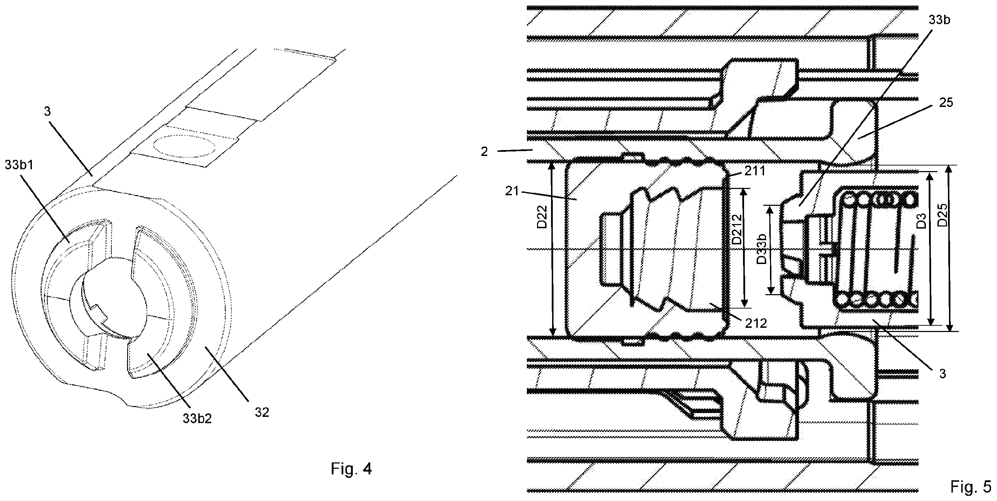

Fig.5 zeigt im Zusammenhang mit einer später beschriebenen zweiten Ausführungsform der Erfindung einen zentralen Ausschnitt aus Fig.1 in vergrösserter Darstellung. Der Produktaufnahmeraum 22 weist einen Innendurchmesser D22 auf, wobei der dargestellte Produktbehälter 2 im Bereich des Flansches 25 einen optionalen Minimalinnendurchmesser D25 aufweist welcher kleiner ist als ein Durchmesser D22 des Produktaufnahmeraums 22. Die Kolbenstange 3 wiederum weist einen Querschnitt mit einem Maximaldurchmesser D3 auf, welcher kleiner ist als der Minimalinnendurchmesser D25 und somit auch kleiner als D22. Der Querschnitt der Kolbenstange 3 kann Ecken umfassen, also beispielsweise viereckig oder dreieckig ausgebildet sein, sollte aber innerhalb eines Kreises Platz finden dessen Durchmesser entsprechend dem Maximaldurchmesser D3 kleiner ist als der Minimalinnendurchmesser D25 des Produktbehälters. Der Kolbenstangenquerschnitt entspricht bevorzugt und zumindest teilweise einem Innenquerschnitt der Kolbenstangenführung 13 (in Fig.5 nicht gezeigt) im proximalen Teil des Gehäuses. Der Produktbehälter 2 weist also in dem von der Kolbenstangenspitze durchschobenen und proximal einer Stopfenendposition liegenden Bereich einen kleinsten Durchmesser auf welcher mindestens vier und bevorzugt mindestens sieben Prozent grösser ist als der Maximaldurchmesser D3 und somit zumindest für eine präzise Längsführung der Kolbenstange nicht geeignet ist. Dafür kann durch die Wahl einer schlanken Kolbenstange im proximalen, an den Flansch 25 anschliessenden Teil des Gehäuses Platz geschaffen werden für zusätzliche konzentrische Komponenten wie beispielsweise die Nadelschutzhülsenfeder 16, oder es können bestehende Komponenten ebenfalls mit kleinerem Durchmesser ausgestaltet werden und somit ein Gesamtdurchmesser des Gerätes reduziert werden.

Durch eine ausschliessliche Führung der Kolbenstage ausserhalb des Produktbehälters ist eine ungenaue oder unzureichende axiale Ausrichtung der Kolbenstange im Produktbehälter nicht mehr vollständig auszuschliessen. Eine Verkippung oder Abweichung von der Längsachse, welche die Kolbenstange nach Einführung in den Produktbehälter in Kontakt mit dem Stopfen noch erleiden kann, ist durch die beteiligten Geometrien auf wenige Grad begrenzt. Trotzdem wird durch eine unsymmetrische Auflage der Kolbenstangenspitze auf den Stopfen die Antriebskraft auf einen Teilbereich der ringförmigen Antriebskraftübertragungsfläche des Stopfens konzentriert und dadurch lokal vergrössert. Insbesondere bei hohen Kräften, beispielsweise bei einem hochviskosen auszuschüttenden Produkt, kann dies zu einer einseitigen Kompression, Quetschung oder gar Verklemmung des Stopfens führen, oder zu einem Ausweichen der Bohrungswand unter Bildung eines Wulstes radial nach innen. Derart unsichere und unvorhersehbare Situationen können schlussendlich zu einer axial nicht genau definierten Endposition der Kolbenstange führen. Als Abhilfe wird an einem distalen Ende der Kolbenstange eine Zentrierhilfe zur Verhinderung der Bewegung der Kolbenstange in Verkippungsrichtung und zur Homogenisierung einer Antriebskraftverteilung über die Auflagefläche vorgesehen. Die Zentrierhilfe kann fest mit der Kolbenstange verrastet, verklebt, verschweisst, verriegelt oder verschnappt sein, oder auch einteilig oder einstückig mit der Kolbenstange ausgebildet sein.

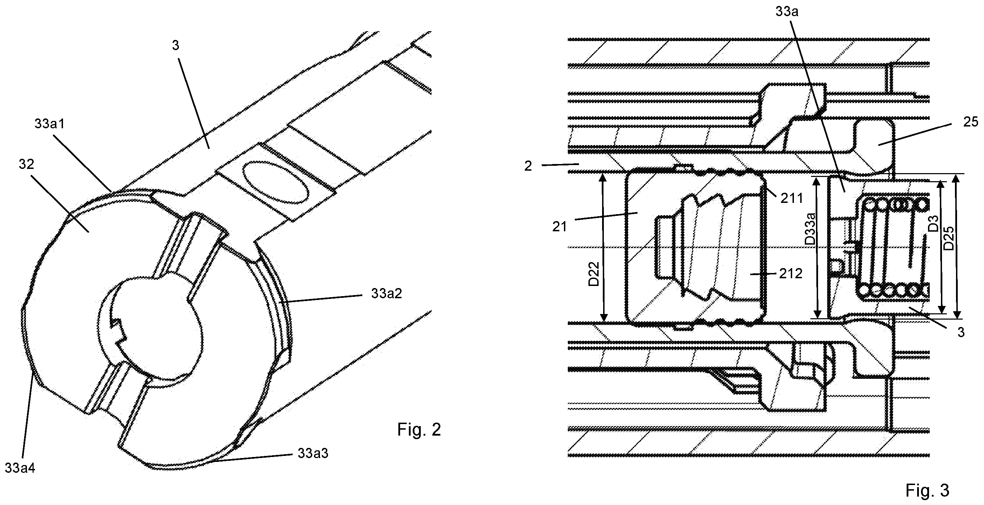

Fig.2 und Fig.3 zeigen eine Schrägaufsicht und einen Längsschnitt einer ersten Ausführungsform der Erfindung mit einer Kolbenstangenzentrier- oder -führungshilfe in Form eines Zentrierflansches 33a, welcher die Kolbenstange 3 seitlich oder radial überragt. Ein Querschnitt des Zentrierflansches 33a senkrecht zur Längsachse hat einen maximalen Flanschdurchmesser D33a oder grösste Linearausdehnung nicht grösser als der Minimalinnendurchmesser D25 des Produktbehälters, aber grösser als der maximale Durchmesser des Kolbenstangenquerschnitts D3. Der Zentrierflansch verbessert die Führung der Kolbenstange im Produktbehälter gegenüber einer Kolbenstangenspitze mit unverändertem Querschnitt.

In einer Variante der ersten Ausführungsform ist aus Herstellungsgründen der Zentrierflansch nicht vollständig umlaufend ausgebildet, sondern in eine Anzahl über den Umfang verteilter, wulstförmiger Segmente mit maximaler radialer Ausdehnung aufgeteilt. In Fig.2 sind vier solcher Flanschsegmente 33a1 bis 33a4 dargestellt, welche untereinander um 90° versetzt angeordnet sind und zudem derart an ihren tangentialen Enden auslaufen, dass sie in einem Quadrat mit einer Seitenlänge gleich dem Kolbenstangendurchmesser D3 Platz finden. Die Antriebsfläche 32 hat die Form eines Kreisringes welcher von einer diagonal verlaufenden Ausnehmung unterbrochen ist. Der dargestellte Zentrierflansch trägt zu einer radialen Vergrösserung der Antriebsfläche 32 bei, er kann alternativ in proximaler Richtung auch um eine kleine Distanz von der Antriebsfläche 32 zurückversetzt sein ohne dadurch seine Zentrierwirkung zu verlieren.

Eine schlanke Kolbenstange ohne Führung im Produktbehälter ist insbesondere bei Produktbehältern mit einem Volumen von über 1 ml und bevorzugt mindestens 2.25 ml und entsprechend angepasstem Minimalinnendurchmesser von Bedeutung. Für eine Fertigspritze mit 2.25 ml Volumen beträgt der Innendurchmesser D22 8.65±0.1 mm und der Minimalinnendurchmesser D25 mindestens 8.25 mm, bei einem Kolbenstangendurchmesser D3 von 7.65±0.1 mm und einer Flanschringbreite von 0.225 mm resultiert ein Flanschdurchmesser D33a von 8.1 mm leicht kleiner als der Minimaldurchmesser D25. Der Durchmesser D13 der Kolbenstangenführung 13 beträgt 7.9±0.1 mm und ist somit deutlich kleiner als der Minimalinnendurchmesser D25 des Produktbehälters.

Fig.4 und Fig.5 zeigen eine Schrägaufsicht und einen Längsschnitt einer zweiten Ausführungsform der Kolbenstangenzentrierhilfe in Form eines Zentrierstumpfs 33b. Der Zentrierstumpf 33b liegt innerhalb der kreisringförmigen Antriebsfläche 32 und weist an seiner Basis einen Basisdurchmesser auf welcher kleiner ist als der Durchmesser D212 der Bohrung 212 des Stopfens 21. Der dargestellte Zentrierstumpf 33b hat gegenüber einem spitzen Kegel oder Konus den Vorteil, dass im Falle eines zu gewährleistenden Minimalabstandes zwischen Stopfen (Auflagefläche 211) und Kolbenstangenspitze im Ausgangszustand die Kolbenstange weniger stark in proximale Richtung verschoben werden muss. Dazu beträgt die Höhe des Zentrierstumpfes bevorzugt weniger als die Hälfte, insbesondere weniger als ein Viertel der Tiefe der Bohrung 212 des Stopfens. Der Zentrierstumpf weist an seiner Spitze einen Stumpfdurchmesser D33b von mindestens einem Viertel, bevorzugt mindestens der Hälfte des Durchmessers D3 der Kolbenstange auf.

An Stelle des dargestellten Kegelstumpfs mit kreisförmiger Basis ist auch ein Pyramidenstumpf mit einer eckigen, insbesondere mindestens viereckigen Grundfläche als Zentrierstumpf möglich. Auch der Zentrierstumpf kann in mehrere Segmente zerfallen, in Fig.4 ist er beispielshaft durch eine diagonale Ausnehmung in zwei Teile 33b1, 33b2 unterteilt. Durch die begrenzte Abweichung von maximal drei Grad gegenüber der Längsachse, unter welcher welche die Kolbenstange nach Einführung in den Produktbehälter auf den Stopfen auftreffen kann, darf auch die Flankensteigung des Zentrierstumpfs, welche nicht kleiner als die genannte Abweichung sein darf, entsprechend bescheiden ausfallen. Die Flanke des Stumpfes dient zur Zentrierung der Kolbenstage bei erstmaligem Kontakt mit dem Rand der Bohrung des Stopfens und die Basis des Stumpfes anschliessend während der Produktausschüttung zur verbesserten Führung der Kolbenstange im Produktbehälter.

In der Patentanmeldung WO2016/205962 ist in Fig.9 ff. ein Autoinjektor beschrieben, umfassend ein Gehäuse mit Längsachse, eine Auslösevorrichtung, einen axial fest im Gehäuse angeordneten Produktbehälter, insbesondere in der Form einer vorgefüllten Spritze oder Karpule, wobei im Produktbehälter ein axial verschiebbarer Stopfen angeordnet ist, durch dessen Verschiebung in distale Richtung Produkt aus dem Produktbehälter ausgeschüttet werden kann. Eine Dreh-, Spiral- oder Triebfeder, in welcher Energie für das automatische Ausschütten von Produkt gespeichert werden kann, ist mit der Auslösevorrichtung gekoppelt, wobei ein erstes Ende der Drehfeder mit dem Gehäuse verbunden ist, und ein zweites Ende der Drehfeder rotationsfest mit einer koaxial zur Längsachse angeordneten Gewindestange verbunden ist. Eine zwei- oder mehrteilige Kolbenstange umfasst mindestens eine äussere, im Gehäuse nicht rotierende Kolbenstange, welche die Form einer Hülse hat und bei einer Verschiebung in distale Richtung den Stopfen des Produktbehälters in distale Richtung mitbewegt. Die zwei- oder mehrteilige Kolbenstange umfasst weiter mindestens eine innere Kolbenstange, welche zumindest teilweise im Inneren der äusseren Kolbenstange gelagert ist und auf diese in distaler Richtung Kräfte übertragen kann. Die innere Kolbenstange weist an ihrem proximalen Ende ein Führungselement auf, welches in Eingriff mit einer Längsführung oder Rippe des Gehäuses sein kann, und ist über eine Gewindeverbindung mit der Gewindestange verbunden, so dass beim Ausschütten eine Drehbewegung der Gewindestange in einer Axialbewegung der inneren Kolbenstange resultiert.

Die äussere und innere Kolbenstange definieren zusammen eine Kavität mit einem inneren Volumen, welches zumindest teilweise mit einem dick- oder zähflüssigen Fluid, beziehungsweise einer gel- oder fettartigen Masse befüllt ist, so dass bei einer relativen Drehung zwischen innerer und äusserer Kolbenstange das dickflüssige Fluid einen Verdrängungswiderstand ausbildet. Das innere Volumen der Kavität kann als Spalt zwischen einem Abschnitt der äusseren Mantelfläche der inneren Kolbenstange und einem Abschnitt der inneren Mantelfläche der äusseren Kolbenstange definiert sein. Alternativ kann die Kavität auch am distalen Ende der inneren Kolbenstange lokalisiert sein und in etwa eine zylindrische Form aufweisen. In beiden Fällen können ruderartige, zumindest mit der inneren Kolbenstange rotationsfest verbundene Fluidverdrängungsstrukturen in die Kavität beziehungsweise in das dickflüssige Fluid hineinragen.

Beim Ausschütten ist die innere Kolbenstange vorerst über das Führungselement drehfest und verschiebbar geführt, erst nach Verschiebung der inneren Kolbenstange über eine gewisse Distanz in distale Richtung wird das Führungselement durch die Längsführung des Gehäuses freigegeben, und die innere Kolbenstange beginnt unter der Wirkung des Drehmoments der Gewindestange zu rotieren. Das dickflüssige Fluid verzögert die Rotation der inneren Kolbenstange sowie möglicher damit drehfest verbundener und im dickflüssigen Fluid eingetauchter Fluidverdrängungsstrukturen. Insbesondere kann die Drehung dieser Strukturen bedingen, dass das dickflüssige Fluid sich aus einer ersten Kammer durch eine Öffnung in eine zweite Kammer zwängen muss, wodurch Druck und Reibung erzeugt werden und die Drehung verlangsamt wird.

Die innere Kolbenstange weist an ihrem proximalen Ende mindestens eine in radiale Richtung ragende Freigabestruktur auf, insbesondere mindestens eine Vertiefung, welche abhängig von ihrer Orientierung zur Längsachse eine freizugebende Struktur freigeben kann. Sobald die verlangsamte Drehung der inneren Kolbenstange eine vorbestimmte Orientierung erreicht hat, wird die Freigabestruktur freigegeben und ein verzögertes Endsignal produziert.

Bevorzugt bildet das proximale Ende der inneren Kolbenstange eine ringförmige Struktur oder einen Flansch mit einem vergrösserten Aussendurchmesser. Diese ringförmige Struktur wird durch eine oder mehrere Ausnehmungen oder Vertiefungen unterbrochen, welche als Führungselement für eine Verdrehsicherung gegenüber der Längsführung des Gehäuses und/oder zur Freigabe des Halteelements nach Abschluss der Produktausschüttung fungieren. Die über den Umfang der ringförmigen Struktur verteilten Ausnehmungen können also je nach Anordnung von Längsführung und Halteelement und den Verzögerungseigenschaften des dickflüssigen Fluids doppelt genutzt werden. Falls beispielsweise das Halteelement im gehaltenen Zustand etwas distal eines distalen Endes der Längsführung und zu dieser um 90° versetzt angeordnet ist kann die innere Kolbenstange zur Verzögerung des Endsignals eine gedämpfte Vierteldrehung vollführen.

Die erfindungsgemäss ausgebildete Kolbenstangenzentrierhilfe kann also auch das distale Ende einer äusseren Kolbenstange sein, welche als Teil eines Verzögerungsmechanismus auf ihrer Innenseite eine Kavität definiert zur Aufnahme eines wie vorgehend beschriebenen und in WO2016/205962 ausführlich offenbarten dickflüssigen Fluids.

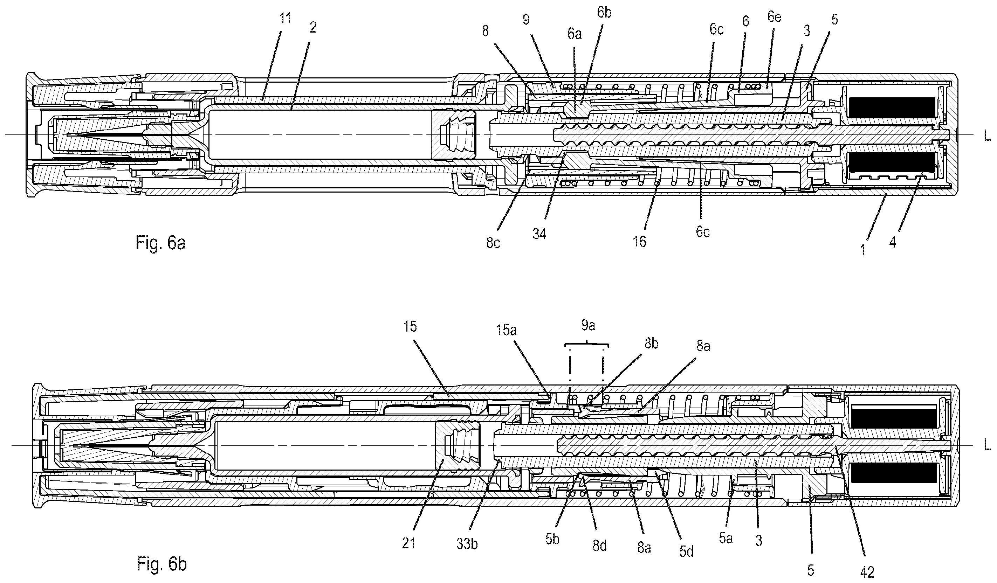

Fig.6a und Fig.6b zeigen zwei um ungefähr 90° versetzte Längsschnittansichten einer weiteren Ausführungsform eines Autoinjektors in einem Auslieferungs- oder Initialzustand. Der Autoinjektor weist ein Halteelement 6 mit zwei Haltearmen 6c auf, wobei an jedem Haltearm 6c ein erstes Eingriffselement 6a und ein zweites Eingriffselement 6b angeordnet sind. Das erste Eingriffselement 6a weist radial zu der Längsachse L hin, wobei das zweite Eingriffselement 6b radial von der Längsachse L weg weist. Das erste Eingriffselement 6a greift in eine Ausnehmung 34, die von der Kolbenstange 3 gebildet wird, ein, wodurch eine Bewegung der Kolbenstange 3 relativ zu dem Halteelement in Ausschüttrichtung verhindert und eine Injektionsfeder 4 oder Ausschüttfeder in Form einer Drehfeder in ihrem gespannten Zustand gehalten wird.

Der Autoinjektor weist ein Schaltmodul 8, 9 auf, welches eine Schalthülse 9 und eine gegenüber der Schalthülse 9 axial bewegbare und von der Schalthülse 9 zumindest teilweise umgebene Sperrhülse 8 aufweist. Das Schaltmodul 8, 9 bildet also einen Teleskopmechanismus mit einer äusseren Teleskophülse 9 und einer inneren Teleskophülse 8. Im Auslieferungszustand der Vorrichtung wird das erste Eingriffselement 6a von dem Innenumfang der Sperrhülse 8, der an dem zweiten Eingriffselement 6b anliegt, in dem Eingriff mit der Ausnehmung 34 gehalten.

Die Schalthülse 9 liegt an einem proximalen Ende 15a der Nadelschutzhülse 15 an. Eine Nadelschutzfeder 16, die vorzugsweise die Schalthülse 9 und die Sperrhülse 8 zumindest teilweise umgibt, stützt sich mit ihrem distalen Ende an der Schalthülse 9 ab. Ein Teil der Schalthülse 9 ist somit zwischen der Nadelschutzhülse 15 und dem distalen Ende der Nadelschutzfeder 16 angeordnet. Die Nadelschutzfeder 16 ist eine als Druckfeder wirkende und als Wendelfeder ausgestaltete Feder aus Metall. Die Nadelschutzfeder 16 stützt sich mit ihrem proximalen Ende ab an einer Abragung 6e des Halteelements welche axial verschiebbar und verdrehfest in das Gehäuse 1 eingreift. Die Nadelschutzfeder 16 umgibt somit auch einen rotations- und axialfest mit dem Gehäuse verbundenen Mechanikhalter 5 zumindest teilweise, vorzugsweise vollständig.

Die Schalthülse 9 weist eine Ausnehmung oder Öffnung9a mit einer axialen Länge oder Ausdehnung L9a auf, welche in axialer Richtung durch eine distale und eine proximale Begrenzung in Form je einer Kante oder eines Rahmenabschnittes begrenzt ist. In die Ausnehmung 9a greift ein sägezahnförmiges und radial von der Längsachse L nach aussen gerichtetes Verriegelungsglied 8b der Sperrhülse 8 von innen ein. Das Verriegelungsglied 8b ist federnd an einem bevorzugt distal orientierten Sperrarm 8a, welcher von der Sperrhülse 8 gebildet wird oder dessen bevorzugt proximales Ende axialfest mit der Sperrhülse 8 verbunden ist, angeordnet. Durch Verschieben der Nadelschutzhülse 15 in die betätigte Position wird die Schalthülse 9 in die proximale Richtung geschoben, dadurch wird auch die Sperrhülse 8 über den Eingriff des Verriegelungsglieds 8b mit einer distalen Begrenzung der Ausnehmung 9a in die proximale Richtung mitgenommen beziehungsweise geschoben. Das Verriegelungsglied 8b kann sich innerhalb und gegenüber der Ausnehmung 9a zwischen der distalen und der proximalen Begrenzung um eine maximale Strecke L9a axial bewegen. Im Initialzustand kann eine Lokalisierungsausnehmung oder Vertiefung 5b im Mechanikhalter 5 dafür sorgen, dass ein Sperrglied 8d auf einer Innenseite des Sperrarmes 8a das Verriegelungsglied 8b innerhalb der Ausnehmung 9a und damit die gesamte Sperrhülse 8 in einer gewünschten axialen Position hält. Die proximale Begrenzung dieser Ausnehmung 5b ist dabei derart ausgebildet und insbesondere angeschrägt, dass das Sperrglied 8d bei einer Betätigungsbewegung in proximale Richtung widerstandsarm daran abgleiten kann. Die Lokalisierungsausnehmung 5b in Fig.6 ist derart axial ausgedehnt, dass eine Bewegung der Sperrhülse 8 im Initialzustand vollständig oder einseitig durch die Ausnehmung 9a in der Schalthülse 9 und das darin eingreifende Verriegelungsglied 8b begrenzt ist, aber nicht beziehungsweise höchstens einseitig durch die Ausnehmung 5b im Mechanikhalter 5. Alternativ dazu kann die Lokalisierungsausnehmung 5b eine minimale axiale Ausdehnung aufweisen und die Sperrhülse punktgenau axial lokalisieren.

Die Sperrhülse 8 weist ein distales Ende 8c auf, welches bei Betätigung oder Aufsetzen des Autoinjektors durch Verschieben der Schalthülse 9 um einen Betätigungshub grösser als L9a entlang der Längsachse L auf die Position des zweiten Eingriffselements 6b gebracht wird. Hierdurch wird das erste Eingriffselement 6a aus dem Eingriff mit der Kolbenstange 3 durch eine Bewegung quer zu und von der Längsachse L weg bewegt, wobei gleichzeitig das zweite Eingriffselement 6b in den Eingriff mit dem distalen Ende 8c der Sperrhülse 8 gelangt. Hierdurch wird die Kolbenstange 3 für die Bewegung um den Ausschütthub in die Ausschüttrichtung freigegeben. Nach erfolgter Auslösung dient der Mechanikhalter 5 als Kolbenstangenführung zur Führung einer Bewegung der Kolbenstange 3 in Richtung der Längsachse. Der starre Querschnitt des Mechanikhalters 5 ist als Führungsquerschnitt Q13 an den Kolbenstangenquerschnitt Q3 angepasst oder darauf abgestimmt und bevorzugt allseitig leicht grösser als letzterer, und begrenzt eine Bewegung des Kolbenstangenquerschnitts in alle Richtungen senkrecht zur Längsachse auf ein fertigungstechnisches Minimum.

Da die axialfeste Kopplung zwischen der Kolbenstange 3 und dem Halteelement 6 nun aufgehoben ist, kann das Halteelement, welches zumindest ein Stück weit relativ zu dem Gehäuse 1 und entlang der Längsachse L bewegbar ist, von der Nadelschutzfeder 16 in die proximale Richtung bewegt werden. Über den Eingriff des zweiten Eingriffselements 6b hinter das distale Ende 8c der Sperrhülse 8 wird diese durch das beschleunigende Halteelement um einen Startsignalhub mitbewegt, bis die Sperrhülse 8 an einem Startsignalanschlag 5a, der von dem Mechanikhalter 5 gebildet wird, anschlägt und hierdurch ein akustisches und/oder taktiles Signal ausgibt, welches dem Anwender der Vorrichtung signalisiert, dass die Produktausschüttung gestartet wurde. Bei dieser Signalbewegung wird die Sperrhülse 8 um weniger als die Länge L9a bewegt, so dass das Verriegelungsglied 8b nicht an der proximalen Begrenzung der Ausnehmung 9a anstösst.

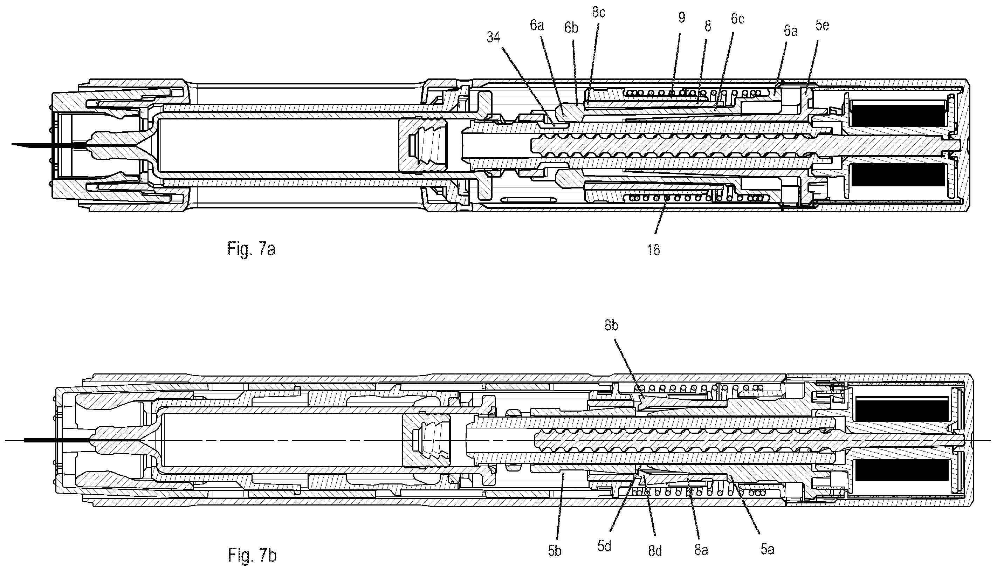

Fig.7a und Fig.7b zeigen zwei um 90° versetzte Längsschnittansichten der weiteren Ausführungsform eines Autoinjektors in einem Zustand nach erfolgter Startsignalisierung und mit aktiviertem Nadelschutz. Durch die Verschiebung der Sperrhülse 8 um den Startsignalhub wird das Sperrglied 8d, welches an Sperrarm 8a der Sperrhülse gegenüber dem Verriegelungsglied 8b angebracht ist und sägezahnförmig radial nach innen beziehungsweise zu der Längsachse L hin ragt, für eine Bewegung quer und zu der Längsachse L hin und in eine Vertiefung 5d des Mechanikhalter 5 freigegeben. Das Sperrglied 8d kann also kurz vor dem Aufschlag der Sperrhülse 8 infolge entsprechender Vorspannung des Sperrarms 8a in die Vertiefung 5d ausweichen und einschnappen, ohne dass dabei das Verriegelungsglied 8b aus der Ausnehmung 9a geraten würde. Dadurch ist die Sperrhülse 8 axial fest mit dem Mechanikhalter 5 verbunden und eine Bewegung der Sperrhülse 8 in die distale Richtung relativ zu dem Gehäuse 1 wird verhindert und ein Nadelschutzmechanismus aktiviert. In dieser Position ist der Sperrarm 8a entspannt, d.h. radial weder gegen innen noch gegen aussen gespannt.

Da das zweite Eingriffsglied 6b immer noch am distalen Ende 8c der Sperrhülse 8 ansteht, wird hierdurch das Halteelement 6 daran gehindert, sich weiter in die proximale Richtung relativ zu dem Gehäuse 1 oder der Sperrhülse 8 zu bewegen. Das zweite Eingriffsglied 6b wird von dem Aussenumfang der Kolbenstange 3 in den Eingriff mit der Sperrhülse 8 gehalten solange die Kolbenstange 3 um ihren Ausschütthub bewegt wird.

Am Ende des Ausschütthubs gibt die Kolbenstange 3 das erste Eingriffsglied 6a für eine Bewegung insbesondere zur Längsachse L hin frei, wodurch das zweite Eingriffsglied 6b aus dem Eingriff mit der Sperrhülse 8 bewegt wird, so dass die Nadelschutzfeder 16 das Halteelement 6 entgegen die Ausschüttrichtung, d. h. in die proximale Richtung beschleunigt, so dass beim Auftreffen des Halteelements 6 auf dem Endsignalanschlag 5e ein akustisches und/oder taktiles Signal erzeugt wird.

Durch Abnehmen des Autoinjektors von der Injektionsstelle kann die Nadelschutzfeder 16 die Schalthülse 9 und die Nadelschutzhülse 15 aus der betätigten Position in die Nadelschutzposition um den Nadelschutzhub bewegen, wobei das Verriegelungsglied 8a aus dem Eingriff mit der Ausnehmung 9a gedrückt wird, wobei sich die Schalthülse 9 relativ zu der Sperrhülse 8 in die distale Richtung bewegt. Wenn die Nadelschutzhülse 15 in ihrer Nadelschutzposition ist, verschnappt das Verriegelungsglied 8a mit der Schalthülse 9, wobei das Verriegelungsglied 8a ein erneutes Zurückschieben der Nadelschutzhülse 15 in ihre betätigte Position verhindert. Bei dem Versuch, die Nadelschutzhülse 15 aus der Nadelschutzposition in die betätigte Position zurückzuschieben, stösst das Schaltglied 9 an dem Verriegelungsglied 8a an, welches die Bewegung der Nadelschutzhülse 15 in die betätigte Position verhindert. Die Sperrhülse 8 stützt sich hierzu axial an dem Startsignalanschlag 5a des Mechanikhalters 5 ab.

### BEZUGSZEICHENLISTE

- 1: Gehäuse
- 11: Produktbehälterhalter
- 12: Endkappe
- 13: Kolbenstangenführung
- 14: Kappenabzugselement
- 15: Nadelschutzhülse
- 15a: Proximales Ende
- 16: Nadelschutzhülsenfeder
- 2: Produktbehälter
- 21: Stopfen
- 211: Auflagefläche
- 212: Bohrung
- 22: Produktaufnahmeraum
- 23: Injektionsnadel
- 24: Nadelschutzkappe
- 25: Flansch
- 3: Kolbenstange
- 31: Abschlusselement
- 32: Antriebsfläche
- 33: Kolbenstangenzentrierhilfe
- 33a: Zentrierflansch
- 33b: Zentrierstumpf
- 34: Ausnehmung
- 4: Injektionsfeder
- 41: Bodenelement
- 42: Gewindestange
- 5: Mechanikhalter
- 5a: Startsignalanschlag
- 5b: Lokalisierungsausnehmung
- 5d: Vertiefung
- 5e: Endsignalanschlag
- 6: Halteelement
- 6a: erstes Eingriffselement
- 6b: zweites Eingriffselement
- 6c: Haltearm
- 6e: Abragung
- 8: Sperrhülse
- 8a: Sperrarm
- 8b: Verriegelungsglied
- 8c: distales Ende
- 8d: Sperrglied
- 9: Schalthülse
- 9a: Ausnehmung

## Patentansprüche

1. Injektionsgerät zur Verabreichung eines flüssigen Produkts, mit
- einem Gehäuse (1) zur Aufnahme eines Produktbehälters (2), in welchem ein Stopfen (21) mit einer Auflagefläche (211) zum Ausschütten des Produkts in Richtung einer Längsachse des Injektionsgeräts bis zu einer Stopfenendposition verschiebbar ist, und welcher proximal der Stopfenendposition einen minimalen Produktbehälterquerschnitt Q25 aufweist,
- einem Antrieb zur Verschiebung des Stopfens (21), umfassend eine Kolbenstange (3) mit einer Antriebsfläche (32) zum Kontakt der Auflagefläche (211) und mit einem Kolbenstangenquerschnitt Q3,
- einer Kolbenstangenführung (13) ausserhalb des Produktbehälters (2) zur Führung einer Bewegung der Kolbenstange (3) in Richtung der Längsachse, mit einem an den Kolbenstangenquerschnitt Q3 angepassten Führungsquerschnitt Q13,
- wobei der Produktbehälter (2) die Bewegung der Kolbenstange (3) in Richtung der Längsachse höchstens unvollständig führt, so dass der Kolbenstangenquerschnitt Q3 zumindest an einer Bewegung in eine Verkippungsrichtung nicht durch den minimalen Produktbehälterquerschnitt Q25 gehindert wird, und
- wobei an einem distalen Ende der Kolbenstange (3) eine Kolbenstangenzentrierhilfe (33a, 33b) zur Begrenzung der Bewegung der Kolbenstange in die Verkippungsrichtung vorgesehen ist,
und wobei die Kolbenstangenzentrierhilfe einen Zentrierflansch (33a) umfasst mit einem maximalen Flanschdurchmesser D33a grösser als ein Maximalaussendurchmesser D3 der Kolbenstange (3) und kleiner als ein Minimalinnendurchmesser D25 des Produktbehälters (2), und wobei der Antrieb weiter eine Injektionsfeder (4) als Antriebsmittel umfasst und die Kolbenstangenführung (13) ein in Richtung der Längsachse verschiebbares Halteelement (6) umfasst, welches eine Bewegung der Kolbenstange (3) in Ausschüttrichtung verhindern und die Injektionsfeder (4) in einem gespannten Zustand halten kann.

2. Injektionsgerät nach Anspruch 1, wobei der minimale Produktbehälterquerschnitt Q25 ein Kreis mit einem Durchmesser D25, der Kolbenstangenquerschnitt Q3 ein Kreis mit einem Durchmesser D3, und der Führungsquerschnitt Q13 ein Kreis mit einem Durchmesser D13 ist, **dadurch gekennzeichnet, dass** D13 kleiner als D25 und grösser gleich D3 ist, und dass D25 mindestens vier Prozent grösser ist als D3.

3. Injektionsgerät nach Anspruch 2, wobei die Auflagefläche (211) des Stopfens (21) ringförmig ist und eine Bohrung (212) im Stopfen (21) umgibt.

4. Injektionsgerät nach Anspruch 1 bis 3, **dadurch gekennzeichnet, dass** der Zentrierflansch aus demselben unelastischen Material gefertigt ist wie die Kolbenstange.

5. Injektionsgerät nach Anspruch 4, **dadurch gekennzeichnet, dass** der Zentrierflansch (33a) die Auflagefläche (211) kontaktiert.

6. Injektionsgerät nach Anspruch 4 oder 5, wobei der Kolbenstangenquerschnitt Q3 in einem Kreis mit einem Durchmesser D3 Platz findet, **dadurch gekennzeichnet, dass** der Zentrierflansch einen Querschnitt aufweist, welcher in ein Quadrat mit einer Seitenlänge D3 passt.

7. Injektionsgerät nach Anspruch 3, **dadurch gekennzeichnet, dass** die Kolbenstangenzentrierhilfe einen Zentrierstumpf (33b) umfasst.

8. Injektionsgerät nach einem der Ansprüche 1 bis 7, **dadurch gekennzeichnet, dass** der Antrieb zur Verschiebung des Stopfens (21) eine gegen ein distales Abschlusselement (31) der Kolbenstange (3) wirkende Druckfeder als Energiespeicher (4) umfasst.

9. Injektionsgerät nach einem der Ansprüche 1 bis 7, **dadurch gekennzeichnet, dass** der Antrieb zur Verschiebung des Stopfens (21) eine Drehfeder umfasst, welche auf eine über eine Gewindeverbindung mit der rotationsfest im Gehäuse (1) gelagerten Kolbenstange (3) verbundene Gewindestange (42) wirkt.

10. Injektionsgerät nach Anspruch 9, **dadurch gekennzeichnet, dass** der Antrieb eine über die Gewindeverbindung mit der Gewindestange (42) verbundene innere Kolbenstange und eine rotationsfest im Gehäuse (1) gelagerte und das Zentriermittel (33b) aufweisende äussere Kolbenstange umfasst, wobei die beiden Kolbenstangen eine mit einem dickflüssigem Fluid befüllbare Kavität bilden zur Dämpfung einer gegen Ende der Ausschüttung freigegebenen Rotationsbewegung der inneren Kolbenstange.

11. Injektionsgerät nach einem der vorangegangenen Ansprüche, **dadurch gekennzeichnet, dass** die Kolbenstangenführung (13) einen axialfest mit dem Gehäuse (1) verbundenen Mechanikhalter (5) umfasst.

12. Injektionsgerät nach einem der vorangegangenen Ansprüche, **dadurch gekennzeichnet, dass** die Injektionsvorrichtung ein Autoinjektor ist und der Produktbehälter eine Fertigspritze mit festmontierter Injektionsnadel und einem nominalen Füllvolumen von 2.25 ml ist.

13. Injektionsgerät nach einem der vorangegangenen Ansprüche, **dadurch gekennzeichnet, dass** das Produkt eine hohe Viskosität von mindestens 5, bevorzugt mindestens 15 cP (0.015 kgm⁻¹s⁻¹) aufweist.

## Claims

1. Injection device for administering a liquid product, comprising
- a housing (1) for receiving a product container (2), in which a plug (21) having a support surface (211) for dispensing the product is displaceable in the direction of a longitudinal axis of the injection device up to a plug end position, and which has a minimum product container cross section Q25 proximal to the plug end position,
- a drive for displacing the plug (21), comprising a piston rod (3) which has a drive surface (32) for contact with the support surface (211) and has a piston-rod cross section Q3,
- a piston rod guide (13) outside the product container (2) for guiding a movement of the piston rod (3) in the direction of the longitudinal axis, which piston rod guide has a guide cross section Q13 matched to the piston-rod cross section Q3,
- wherein the product container (2) guides the movement of the piston rod (3) in the direction of the longitudinal axis at most incompletely, so that the piston-rod cross section Q3 is at least not prevented from moving in a tilting direction by the minimum product container cross section Q25, and
- wherein a piston-rod centering aid (33a, 33b) is provided at a distal end of the piston rod (3) to limit the movement of the piston rod in the tilting direction,
and wherein the piston rod centering aid comprises a centering flange (33a) having a maximum flange diameter D33a greater than a maximum outer diameter D3 of the piston rod (3) and smaller than a minimum inner diameter D25 of the product container (2), and wherein the drive further comprises an injection spring (4) as the drive means and the piston rod guide (13) comprises a holding element (6) which is displaceable in the direction of the longitudinal axis, can prevent movement of the piston rod (3) in the dispensing direction, and can hold the injection spring (4) in a tensioned state.

2. Injection device according to claim 1, wherein the minimum product container cross section Q25 is a circle having a diameter D25, the piston-rod cross section Q3 is a circle having a diameter D3, and the guide cross section Q13 is a circle having a diameter D13, **characterized in that** D13 is smaller than D25 and greater than or equal to D3, and **in that** D25 is at least four percent greater than D3.

3. Injection device according to claim 2, wherein the support surface (211) of the plug (21) is annular and surrounds a bore (212) in the plug (21).

4. Injection device according to claims 1 to 3, **characterized in that** the centering flange is made of the same inflexible material as the piston rod.

5. Injection device according to claim 4, **characterized in that** the centering flange (33a) makes contact with the support surface (211).

6. Injection device according to claim 4 or 5, wherein the piston-rod cross section Q3 fits into a circle having a diameter D3, **characterized in that** the centering flange has a cross section that fits into a square having a side length D3.

7. Injection device according to claim 3, **characterized in that** the piston rod centering aid comprises a centering projection (33b).

8. Injection device according to any of claims 1 to 7, **characterized in that** the drive for displacing the plug (21) comprises as an energy store (4) a compression spring acting against a distal terminal element (31) of the piston rod (3).

9. Injection device according to any of claims 1 to 7, **characterized in that** the drive for displacing the plug (21) comprises a torsion spring which acts on a threaded rod (42) which is connected via a threaded connection to the piston rod (3) mounted in the housing (1) in a rotationally fixed manner.

10. Injection device according to claim 9, **characterized in that** the drive comprises an inner piston rod, which is connected via the threaded connection to the threaded rod (42), and an outer piston rod, which is mounted in the housing (1) in a rotationally fixed manner and has the centering means (33b), wherein the two piston rods form a cavity that can be filled with a viscous fluid to attenuate a rotational movement of the inner piston rod, which movement is allowed towards the end of the dispensing.

11. Injection device according to any of the preceding claims, **characterized in that** the piston rod guide (13) comprises a mechanism holder (5) which is connected to the housing (1) in an axially fixed manner.

12. Injection device according to any of the preceding claims, **characterized in that** the injection apparatus is an auto-injector and the product container is a pre-filled syringe having a fixed injection needle and a nominal filling volume of 2.25 ml.

13. Injection device according to any of the preceding claims, **characterized in that** the product has a high viscosity of at least 5, preferably at least 15 cP (0.015 kgm⁻¹s⁻¹).

## Revendications

1. Appareil d'injection pour l'administration d'un produit liquide, comportant
- un boîtier (1) permettant de recevoir un récipient pour produit (2) dans lequel un bouchon (21) comportant une surface d'appui (211) pour la distribution du produit peut être déplacé dans la direction d'un axe longitudinal de l'appareil d'injection jusqu'à une position d'extrémité de bouchon et qui présente une section transversale de récipient pour produit minimale Q25 proximale à la position d'extrémité de bouchon,
- un entraînement pour le déplacement du bouchon (21), comprenant une tige de piston (3) comportant une surface d'entraînement (32) pour le contact avec la surface d'appui (211) et comportant une section transversale de tige de piston Q3,
- un guidage pour tige de piston (13) à l'extérieur du récipient pour produit (2) permettant de guider un mouvement de la tige de piston (3) dans la direction de l'axe longitudinal, comportant une section transversale de guidage Q13 adaptée à la section transversale de tige de piston Q3,
- dans lequel le récipient pour produit (2) guide tout au plus incomplètement le mouvement de la tige de piston (3) dans la direction de l'axe longitudinal, de sorte que la section transversale de tige de piston Q3 n'est pas bloquée par la section transversale de récipient pour produit minimale Q25, au moins lors d'un mouvement dans une direction de basculement, et
- dans lequel un moyen d'aide de centrage de tige de piston (33a, 33b) est prévu à une extrémité distale de la tige de piston (3) pour limiter le mouvement de la tige de piston dans la direction de basculement,
et dans lequel le moyen d'aide au centrage de tige de piston comprend une bride de centrage (33a) d'un diamètre de bride maximal D33a supérieur à un diamètre extérieur maximal D3 de la tige de piston (3) et inférieur à un diamètre intérieur minimal D25 du récipient pour produit (2), et dans lequel l'entraînement comprend en outre un ressort d'injection (4) comme moyen d'entraînement et le guidage de tige de piston (13) comprend un élément de retenue (6) pouvant être déplacé dans la direction de l'axe longitudinal et pouvant empêcher un mouvement de la tige de piston (3) dans la direction de distribution et maintenir le ressort d'injection (4) dans un état tendu.

2. Appareil d'injection selon la revendication 1, dans lequel la section transversale de récipient pour produit minimale Q25 est un cercle d'un diamètre D25, la section transversale de tige de piston Q3 est un cercle d'un diamètre D3, et la section transversale de guidage Q13 est un cercle d'un diamètre D13,
**caractérisé en ce que** D13 est inférieur à D25 et supérieur ou égal à D3,
**et en ce que** D25 est au moins quatre pour cent plus grand que D3.

3. Appareil d'injection selon la revendication 2, dans lequel la surface d'appui (211) du bouchon (21) est annulaire et entoure un alésage (212) dans le bouchon (21).

4. Appareil d'injection selon les revendications 1 à 3,
**caractérisé en ce que** la bride de centrage est fabriquée à partir du même matériau non élastique que celui de la tige de piston.

5. Appareil d'injection selon la revendication 4, **caractérisé en ce que** la bride de centrage (33a) est en contact avec la surface d'appui (211).

6. Appareil d'injection selon la revendication 4 ou 5, dans lequel la section transversale de tige de piston Q3 se situe dans un cercle d'un diamètre D3, **caractérisé en ce que** la bride de centrage présente une section transversale qui s'inscrit dans un carré d'une longueur de côté D3.

7. Appareil d'injection selon la revendication 3, **caractérisé en ce que** le moyen d'aide au centrage de tige de piston comprend un cône de centrage (33b).

8. Appareil d'injection selon l'une des revendications 1 à 7,
**caractérisé en ce que** l'entraînement pour le déplacement du bouchon (21) comprend comme accumulateur d'énergie (4) un ressort de compression agissant contre un élément terminal distal (31) de la tige de piston (3).

9. Appareil d'injection selon l'une des revendications 1 à 7,
**caractérisé en ce que** l'entraînement pour le déplacement du bouchon (21) comprend un ressort de torsion qui agit sur une tige filetée (42) reliée par l'intermédiaire d'une liaison par filetage à la tige de piston (3) montée de manière fixe en rotation dans le boîtier (1).

10. Appareil d'injection selon la revendication 9, **caractérisé en ce que** l'entraînement comprend une tige de piston intérieure reliée à la tige filetée (42) par l'intermédiaire de la liaison par filetage et une tige de piston extérieure montée de manière fixe en rotation dans le boîtier (1) et présentant le moyen de centrage (33b), dans lequel les deux tiges de piston forment une cavité pouvant être remplie d'un fluide visqueux pour amortir un mouvement de rotation de la tige de piston intérieure libéré vers la fin de la distribution.

11. Appareil d'injection selon l'une des revendications précédentes, **caractérisé en ce que** le guidage de tige de piston (13) comprend un support mécanique (5) relié de manière fixe axialement au boîtier (1).

12. Appareil d'injection selon l'une des revendications précédentes, **caractérisé en ce que** le dispositif d'injection est un auto-injecteur et le récipient pour produit est une seringue prête à l'emploi comportant une aiguille d'injection montée de manière fixe et un volume de remplissage nominal de 2,25 ml.

13. Appareil d'injection selon l'une des revendications précédentes, **caractérisé en ce que** le produit présente une viscosité élevée d'au moins 5, de préférence d'au moins 15 cP (0,015 kgm⁻¹s⁻¹).
